# EUROPEAN PATENT APPLICATION

(11) **EP 1 681 068 A1**
(43) Date of publication of application: **19.07.2006**
(21) Application number: 04029796.2
(22) Date of filing: 16.12.2004
(51) Int. Cl.: A61M 1/36, A61L 15/00

(54) **Anti-pathogen and anti-cancer filtering device and method comprising nitric oxide**

(71) Applicant: NOLabs AB, 252 21 Helsingborg (SE)
(72) Inventor: Peters, Tor, 8703 Erlenbach (CH)
(74) Representative: Petri, Stellan

(57) **Abstract**

A filtering device and method are disclosed being adapted to eliminate, inactivate and/or remove pathogenic or cancerous elements, especially microorganisms, such as bacteria, parasites, fungi, mycoplasma, protozoa and viruses, or tumour cells from fluids, especially body fluids or fluids getting into contact with the body, by using NO originating from the filtering device material. Exemplary embodiments comprise blood filters, or breathing gas filters.

## Description

### Field of the Invention

This invention pertains in general to the field of medical fluid treatment. More particularly the invention relates to filters for medical devices used to effectively eliminate, inactivate and/or remove pathogenic and/or cancerous elements, especially microorganisms, such as bacteria, parasites, fungi, mycoplasma, protozoa, and viruses, or cancer / tumour cells, from fluids, especially body fluids or fluids getting into contact with the body.

### Background of the Invention

In a variety of circumstances, pathogenic elements, especially microorganisms, such as bacteria, parasites, as e.g. malaria, protozoa and viruses, and/or cancerous elements, such as tumour cells, e.g. leukaemia, must be eliminated, inactivated and/or removed from body fluids such as blood or respiratory gases. This is generally done for the purpose of treatment of a disease related to said pathogenic and/or cancerous elements. Often, when a disease related to the elements is already present in a patient, the elimination, inactivation and/or removal is done in order to reduce the number of the elements in the body, so that a medical treatment is facing less antagonists, and becomes thus more effective. Alternatively, the elements in question are hindered from entering or leaving a body in order to reduce the risk for transferring contagious or infectious elements to or from a body.

Membrane filtration is one known way of removing such pathogenic elements, wherein particles are separated by size exclusion based on a sieving principle. Therefore, filtration is limited to the removal of such pathogenic elements by size irrespective of chemical or thermal characteristics thereof.

Furthermore, various methods of inactivating pathogenic or cancerous elements in fluids are known, e.g. heat treatment, radiation treatment or chemical treatment methods. However, the heat treatment method exerts little effect on certain heat-resistant pathogenic elements, e.g. human parvovirus B19, hepatitis A virus, and the like. Moreover, known chemical treatment methods have essentially no effect on certain types of pathogenic elements, e.g. human parvovirus B19, poliovirus, reovirus, and SV-40 having no lipid envelope. In particular, the virus removal membrane is, as of today, in certain cases the only effective device for inactivating or removing pathogens. This is for instance the case for the human parvovirus B19, which is both heat resistant and has no lipid envelope. Chemical, and especially radiation treatment has often unwanted vigorous side effects.

Removal membranes for pathogenic elements available at present are either based on a membrane, which allows high-molecular-weight physiologically active products such as human immunoglobulin and Factor VIII to pass there through, but exhibits inferior small virus removal performance, or a membrane which can remove small viruses, but cannot allow high-molecular-weight physiologically active products such as human immunoglobulin or Factor VIII to pass there through at a practical level.

Hence, there are no adequate therapies available for a number of diseases where for instance blood carried pathogenic elements, such as viruses, or cancerous elements play a central role. Moreover, an increasing number of viruses and the currently ongoing mutations of e.g. bird flu viruses create a need for innovative ways of effective treatment. Hence, an alternative and/or improved way of at least partly eliminating, inactivating and/or removing pathogenic elements or cancerous elements from body fluids or fluids getting into contact with the body would be advantageous.

### Summary of the Invention

Accordingly, the present invention preferably seeks to mitigate, alleviate or eliminate one or more of the above-identified deficiencies in the art and disadvantages singly or in any combination and solves at least the above mentioned problems by providing a filtering device, a manufacturing process therefor, and a method, according to the appended patent claims.

According to one aspect of the invention, a filtering device adapted to eliminate, inactivate and/or remove pathogenic and/or cancerous elements from fluids, especially body fluids or fluids getting into contact with the body, is provided. The device comprises a nitric oxide (NO) eluting polymer arranged to contact said fluid when in use of said filtering device passing there through, such that a therapeutic dose of nitric oxide is eluted from said nitric oxide eluting polymer to said fluid.

According to another aspect of the invention, a manufacturing process for such a filtering device is provided, wherein the process is a process for forming a filtering device adapted to eliminate, inactivate and/or remove pathogenic elements, especially microorganisms, such as bacteria, parasites, fungi, mycoplasma, protozoa and viruses, or cancerous elements from fluids. The process comprises selecting a plurality of nitric oxide eluting polymeric fibers, and deploying said nitric oxide eluting fibers to be comprised in a filter membrane.

According to yet another aspect of the invention, a method for eliminating, inactivating and/or removing pathogenic elements, especially microorganisms, such as bacteria, parasites, mycoplasma, protozoa and viruses, or cancerous elements from fluids, is provided. The method comprises providing a filtering device comprising a nitric oxide eluting polymer, and eluting nitric oxide from the polymer upon contact with the fluid for achieving an anti-pathogenic and/or anti-cancerous effect.

The present invention has at least the advantage over the prior art that it provides exposure of particles comprised in a fluid, e.g. blood particles or any other particles contained in blood, to be homogeneously exposed to NO, whereby a very effective anti-viral, anti-bacterial, anti-fungi and/or anti-cancer therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of embodiments of the invention with reference to a patient;
Fig. 2 is a schematic illustration of a fibre filtering layer comprised in an embodiment of the invention;
Fig. 3 is a schematic illustration of a multi-layer blood filter according to an embodiment of the invention;
Fig. 4 is a schematic illustration of a breathing gas filter according to another embodiment of the invention; and
Fig. 5 is a schematic illustration of another breathing gas filter according to another embodiment of the invention.

### Description of embodiments

The following description focuses on different embodiments of the present invention applicable to an apparatus treating human blood whilst outside the body, respiratory filters, e.g. a breathing mask, and other types of filtering devices. However, it will be appreciated that the invention is not limited to these applications mentioned in the different embodiments, but may be applied to many other similar areas where fluids are filtered, including for example water purification systems, purification systems used within the pharmaceutical industry, air filtration systems, etc.

However, all embodiments have in common that Nitric Oxide is used, taking advantage of its anti-pathogenic and anti-cancerous effect.

### Nitric Oxide

Hitherto Nitric Oxide (NO) has been used for improving healing processes of implanted medical devices. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral infections with NO and it is difficult if not impossible until now to expose larger amounts of body fluids to NO. This is because NO is actually toxic and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

The applicants of the present invention's new technology build on the concept of reducing the amount of fluid, e.g. blood or respiratory gas, carried pathogenic elements, e.g. viruses, bacteria, fungi, mycoplasma, cancerous elements etc. This is established by exposing the fluid, e.g. blood comprising blood cells, to NO by means of an extracorporeal "filter" device. The "filter" device is fully or partly consisting of polymer filaments eluting NO when in contact with the body fluid. In this way, an anti-bacterial, anti-viral, anti-fungi, anti-cancer, etc. element is provided for reducing pathogenic and/or cancerous elements, especially microorganisms, such as bacteria, parasites, fungi, protozoa, and viruses, or cancer / tumour cells, from fluids.

The expression "filter" in this context is directed towards a porous article or mass, as of cloth, paper, polymeric material, metal, etc., that serves as a medium for establishing a contact with a liquid or gas passed through it, so that matter held in suspension or dissolved in the liquid or the gas may be separated thereof. However, the main purpose of the "filter" for the present invention is to expose the liquid or gas to nitric oxide supplied by means of the "filter", rather than filtering out the pathogenic elements or matter. This means that the pathogenic and/or cancerous elements are at least to be immobilised or rendered innocuous by the "filter", and the possibility of binding these immobilised, i.e. no longer pathogenic or carcinogenic, elements to the filter is only a secondary possibility offered by the nature of the filtering device, which is advantageous for certain applications.

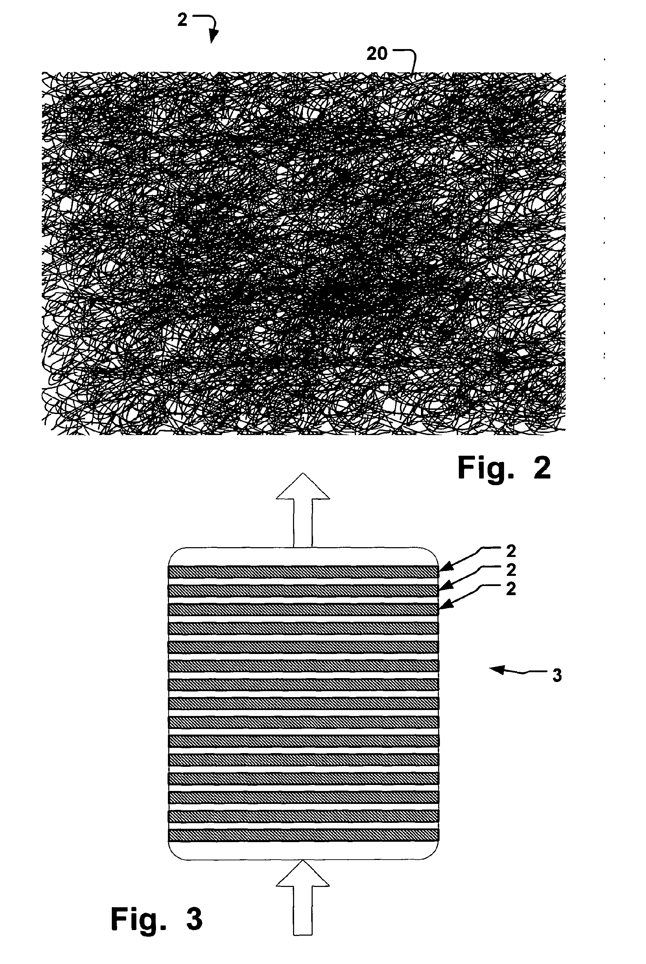

The filter device comprises a high number of filaments, which allows a high number of blood molecules to be exposed to NO on an individual basis. An exemplary, nonlimiting filament arrangement is shown in Fig. 2. The filter device may have the NO eluting polymer coated onto its filter membrane or it may comprise the polymer as a structural material through which the fluid to be treated passes in use of the filter. The polymer may be any suitable material capable of storing NO in a sufficient amount. The filtering device comprising the polymer shall be storable over a longer time without a substantial loss of stored NO, so that it is fully functional at the time of use. A preferred material is L-PEI, explained in more detail below. Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups. Other examples disclosed are S-nitrosylated .beta.-cyclodextrin or an S-nitrosylated .beta.-cyclodextrin complexed with S-nitroso-N-acetyl-D,L-penicillamine or S-nitroso-penicillamine, S-nitrosylated .beta.-cyclodextrin and S-nitrosylated .beta.-cyclodextrin complexed with S-nitroso-N-acetyl-D,L-penicillamine. Compositions comprising S-nitrosylated cyclodextrins complexed with S-nitrosothiols, have been found to deliver NO-related activity for extended periods of time and to exhibit good shelf stability.

Mixtures of suitable polymers may also be used for storing NO in filtering devices.

According to one embodiment, the filter device is comprised in an improved oxygenator assembly, currently used in open heart surgery. According to another embodiment, the filter device is comprised in an artificial kidney assembly, as currently used in dialysis.

Adding NO eluting technology, preferably nano-fiber technology of L-PEI, converts existing filtering devices into therapeutic devices.

One of the advantages of the NO-eluting nano-fiber technology is a constant and non-toxic elution of NO into the body fluid and exposure of all naturally occurring cells, virus and other particles e.g. bacteria to NO.

Toxicity is avoided by a combination of local NO elution and the short half-life of NO.

Exposure and contact between particles suspended in body fluids and NO is guaranteed by the presence of a large number, e.g. some millions, of NO-eluting nano-fibers.

This technology, for instance applied in a blood filtering device, significantly reduces the amount of active blood carried virus such as Hepatitis, HIV, Ebola and Influenza allowing drug therapy to become more effective treating residuals.

### Linear poly(ethylenimine) L-PEI

Akron University has developed NO-eluting L-PEI molecule that can be nano-spun onto the surface of medical devices such as implanted grafts, showing significant improvement of the healing process and reduced inflammation when implanting such devices. This is for instance described in US-6,737,447. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning a filtering application of body fluids, and the anti pathogenic potential of nitric oxide.

According to embodiments of the invention described below, such nanofibers, preferably electrospun onto filter devices or electrospun into filter devices, are used for achieving an anti-pathogenic and/or cancerous effect when "filtering" body fluids or fluids getting into contact with the body.

For this purpose, L-PEI fibres are preferably produced by the technique of electrostatic spinning, also known within the fiber forming industry as electrospinning, of liquids and/or solutions capable of forming fibers. Electrospinning is well known and has been described in a number of patents as well as in the general literature. The process of electrostatic spinning generally involves the introduction of a liquid into an electric field, so that the liquid is caused to produce fibers. These fibers are generally drawn to a cathode for collection. During the drawing of the liquid, the fibers harden and/or dry. This may be caused by cooling of the liquid, i.e., where the liquid is normally a solid at room temperature; by evaporation of a solvent, e.g., by dehydration (physically induced hardening); or by a curing mechanism (chemically induced hardening).

One of the major advantages of using electrostatically spun fibers is that these fibers may be produced having very thin diameters, usually on the order of about 100 nanometers to about 25 microns, and more preferably, on the order of about 100 nanometers to about 1 micron. Thus, these fibers may be collected and formed into coatings for filters or non-woven membranes of filter devices of any desired shape and thickness. It will be appreciated that, because of the very small diameter of the fibers, the resultant coating or membrane has a very small interstices and high surface area per unit mass, which is an advantage for filtering applications.

US-6,737,447 discloses a method for the production of fibers of linear poly(ethylenimine) diazeniumdiolate using electrospinning techniques. Such fibers have very small diameters of less than 1 micron, and, more preferably, less than 400 nanometers. The fibers also have very high surface areas per unit mass and are capable of releasing therapeutic levels of NO as needed. These fibres are highly effective in delivering NO in combination with implanted devices, towards tissues surrounding medical devices while minimizing the alteration of the properties of the devices. The same applies to the embodiments of the present invention, wherein these fibres are effectively delivering NO to surrounding fluids flowing through a filter comprising the fibres.

This effectiveness of NO delivery is for instance accomplished by using electrostatically spun nanofibers of polymeric NONOate to coat the filter membrane of the filtering device. A nanofiber coating, because of the small size and large surface area per unit mass of the nanofibers, provides a much larger surface area while minimizing changes in other properties of the filter device. Nanofibers of poly(ethylenimine)diazeniumdiolate are preferably used for medical filtering devices according to the embodiments describe below. Alternatively, nanofibres 20 are spun into filter elements 2, such as filtering pads or sheets. Multiple such sheets may be arranged in a suitable way, e.g. piled up (as shown in Fig. 3) or folded to multiple layers, so that an effective contact of the fluid flowing through the filter device is achieved. Alternatively a plurality of filters may be used subsequently.

The aforementioned poly(ethylenimine)-diazeniumdiolate fibers release NO with a half-life in the range of 6-30 hours at pH 7.4 and 37[deg.] C. Once released, NO will contribute with its anti-pathogenic, anti-viral, anti-bacterial, anti-fungi and/or anti-cancerous effect.

The release of NO from this polymer is humidity controlled. Thus, activation is triggered by e.g. body fluids, such as blood, sweat or, if available in an sufficient amount, air humidity. Hence, filtering devices may be activated to release NO when taken out of a suitable humidity less storage environment, such as plastic package having a defined low or no humidity atmosphere inside, and when getting into contact with sufficient activation humidity. This occurs e.g. when getting into contact with a body liquid on which the NO is to be employed, wherein this is for instance blood or respiratory gases to be treated; expiratory gas has e.g. 100% relative humidity at body temperature.

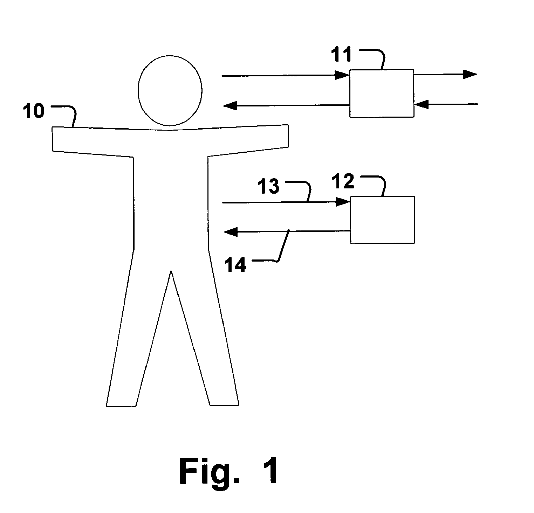

As shown in Fig. 1, a patient 10 may benefit from such filter devices 11, 12 comprising NO eluting polymers in several ways. One possibility is to filter breathing gases, both entering or leaving the body, by means of filter device 11. Another possibility is to take a body fluid, such as blood, feed it to filter device 12, and return it to the body after treatment with NO in device 12. Below, a number of embodiments are given in order to illustrate these principles.

### Extracorporeal blood filtering

An embodiment of the invention pertaining to extracorporeal blood filtering is described below with reference to Figs. 1, 2 and 3.

Numerous techniques have been developed for circulating blood of a patient outside the body in an "extracorporeal" circuit and then returning it to the patient, for instance during a surgical procedure or during a dialysis. For example, in dialysis for patients with kidney failure, blood is circulated extracorporeally and contacted with a large membrane surface separating the blood from a dial sate solution, and urea and other blood chemicals are migrated across the membrane to cleanse the blood, which is then returned to the patient. In ex vivo organ perfusion, such as liver perfusion for patients with liver failure, blood is circulated extracorporeally and per fused through a donor organ, typically a pig liver in the case of liver perfusion, before returning it to the patient. In cases of thermal treatment, blood is circulated out of the body and through a heat exchanger and returned to the body. In heart surgery, either or both ventricles of the heart may be isolated and surgically repaired while making use of the patient's lungs during the surgery. In left monoventricular surgery, the left ventricle is isolated for surgery by cannulating the left atrium into an extracorporeal circuit, which pumps the blood into a cannulated femoral artery or other arterial source to the arterial bed. In biventricular surgery, the right ventricle is isolated for surgery by cannulating the right atrium and feeding the blood extracorporeally to the pulmonary artery, and the left ventricle is isolated by cannulating the left atrium and feeding the oxygenated blood extracorporeally to a femoral or other artery for perfusion of the arterial bed.

Another example of extracorporeal circulation is cardiopulmonary bypass ("CPB"), the procedure of mechanically bypassing both the heart and lungs to allow the whole heart to be isolated for surgical repair. A CPB machine, consisting of a number of independent and discrete components linked together by plastic tubing, assumes the function of the heart and lungs by oxygenating the blood of the patient, returning the oxygenated blood to the body, and pumping it through patient's circulatory system. More particularly, in CPB the patient's inferior and superior venae cava are cannulated and the blood is ducted from the patient to a venous reservoir in the CPB circuit. From the venous reservoir, this circuit then connects to a pump, which circulates the blood. The blood is then oxygenated by being pumped through a gas exchange reservoir, i.e. the above-mentioned oxygenator, where oxygen is added and carbon dioxide is removed from the system. The next CPB element is the heat exchanger where the temperature of the blood can be altered and controlled. This device is typically coupled in parallel to the oxygenator. The last element of the extracorporeal circuit is typically a filter used to eliminate particulate matter accumulated in the extracorporeal system. The oxygenated blood then re-enters the body of the patient through another cannula in the arterial system. Other elements which are part of the CPB system but operated in parallel to the circuit include systems used to retain suctioned blood in the operative field to return to the patient ("cardioplegia") and systems to filter and concentrate the cells also to be given to the patient through the CPB circuit (cell savers or hemoconcentrators).

The filtering device 3 according to an embodiment of the invention is used for eluting NO into blood of a patient 10, during one of the above-mentioned or similar treatments in which blood is conducted out of the patient body. Alternatively, the filtering device 3 may be used in a "stand-alone" circuit, solely for taking advantage of the anti-pathogenic and/or cancerous effect, without additional therapy, such as oxygenation or dialysis.

According to a more detailed embodiment, the filter device is incorporated in an extracorporeal blood circulation apparatus. The apparatus comprises an inlet line 13 adapted to receive blood from a patient, an outlet line 14 adapted to return blood to the patient, a fluid circuit for fluid communication between the inlet and the outlet line, at least one pump acting on the fluid circuit to circulate blood there through and out the outlet line, and one or more nitric oxide eluting filters 3, 12 between the inlet and the outlet. Such a blood fluid circuit includes for instance the monoventricular and biventricular bypass circuits described above. The fluid circuit may also comprise a blood treatment portion such as a dialysis component, an organ perfusion component, a heat exchange component or an oxygenation component for blood treatment as discussed above.

Because of the very short half-life of nitric oxide in blood, a nitric oxide concentration in the circulating extracorporeal blood is achieved at a dosage effective to produce the desired anti-pathogenic or anti-cancerous effect in the extracorporeal circuit without harming the patient, due to the above mentioned vasodilatory effect of NO. Preferably the blood velocity in the extracorporeal circuit is adjusted in such a way that the NO concentration in the blood has a sufficiently low level to not cause harm to the patient after the blood is returned to the patient.

The level of NO administered to the blood is controlled by the amount of NO being able to elute from the NO eluting polymer of the filter device, preferably L-PEI, and by the rate of blood flow passing the filter device. With a known volume of the filter device, the NO concentration at the filter device may be calculated. For instance, a differential pressure sensor may be used to calculate the blood flow rate over the filter device according to known techniques. In combination with the polymer characteristics, the volume of the filter and the blood flow rate, the NO concentration and a distribution over time and the length of the extracorporeal circuit is established and may be used for controlling the NO level by controlling the pump of the extracorporeal circuit. The extracorporeal circuit is also compatible with pulsatile blood flows.

According to the present embodiment, a known cardiopulmonary bypass machine is improved by adding anti-pathogenic and/or cancerous functionality to it.

Another application of such a blood filter is during blood donation or transfusion. While the blood bank community has successfully implemented the higher standards of viral, retroviral for reducing such pathogens in cellular blood components, transmission of donor bacteria is still a great infectious risk to transfusion safety. A bacterial sepsis is regarded to be the most frequent transfusion-associated infectious complication. By using a filter device according to the above embodiment, bacteria in donated blood are inactivated, eliminated and/or removed shortly after collection. Alternatively, the filtering device may be used for this purpose during blood transfusion into the body.

It is known to apply leukoreduction by filtration for the blood bank. Therefore, the NO eluting polymer is according to another embodiment applied to such a leukocyte filtration device in order to further reduce the likelihood of bacterial proliferation in red cell and platelet components. Such leukocyte reduction filters effectively reduce or remove certain bacteria from blood components.

Platelet concentrates, stored at room temperature, are most vulnerable to bacterial growth. When process improvements cannot completely eliminate the unintended entry of bacteria into blood products, the present filtering devices may be the only way of reducing infectious risks.

Another embodiment of the invention is now described with reference to embolism filters. For instance the Pall® AutoVent™ SV Blood Filter for Extracorporeal Service may be provided with NO eluting properties. This filter is designed to remove microemboli greater than 40 µm in size from perfusate during extracorporeal circulation. This includes gas emboli, fat emboli and aggregates composed of platelets, red blood cells and other debris. This filter automatically separates and vents gas emboli that may be generated by pressure drops, temperature changes or oxygen oversaturation of blood. In combination with a filtering device having NO eluting polymers, or by coating the existing AutoVent™ SV Blood Filter with an NO eluting polymer, embolism filters are considerably improved with the anti-pathogenic and/or anti-cancerous properties.

### Gas filtering, e.g. respiratory gas filtering

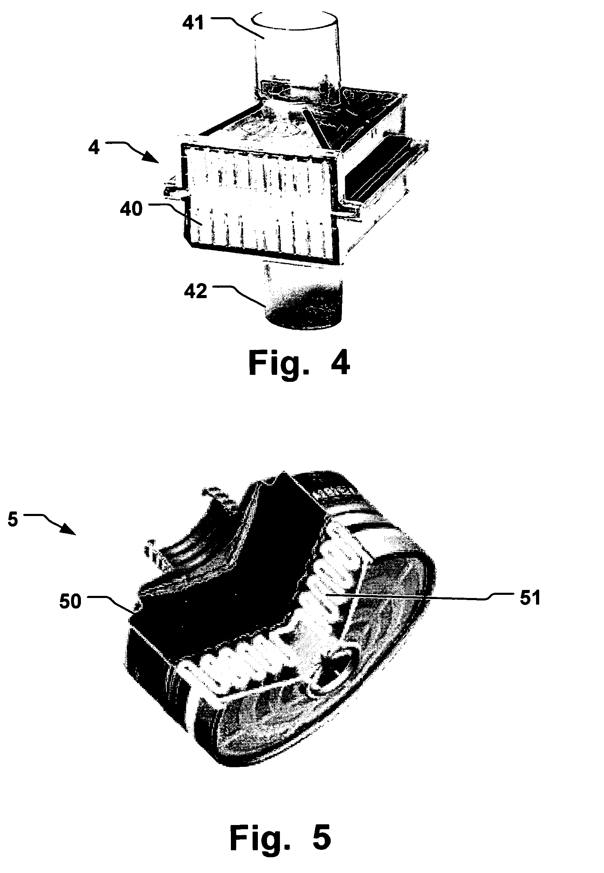

According to the embodiment described below, the NO eluting filtering media is applied in gas filtration. Embodiments, related to respiratory gas filtering are illustrated with reference to Figs. 4 and 5.

In this way, biological pathogens like anthrax, small pox and the like are treated by the NO eluting from a polymer comprised in a filter. The filter media of the present embodiment are thus able to provide such protection when e.g. incorporated into a replaceable filter cartridge of a gas mask.

Medical procedures in which patients share life-saving devices pose an especially high potential risk of cross infection. This may be problematic with prions due to their long incubation period where there are no signs or symptoms of disease and no tests to determine their presence in humans. It is current practice in certain anaesthesia locales to reuse the breathing circuit between patients, when a filter is used at the patient end.

Hence, a further field of application for embodiments of the invention is respiratory care.

Prion diseases are fatal, neurodegenerative diseases, referred to as Transmissible Spongiform Encephalopathies (TSEs), that affect both humans and animals. They include scrapie in sheep, bovine spongiform encephalopathy in cattle and variant CJD in humans. It is estimated that the incubation period (prior to clinical symptoms) for variant CJD may be anywhere between 10 to 20 years. The first patient death from variant Creutzfeldt-Jacob Disease (vCJD, the human form of "mad cow disease") as a possible result of a blood transfusion has raised new concerns about the potential transmission of prions during other medical procedures. One area of concern is in the use of respiratory equipment between patients during anaesthesia procedures. Studies show that up to 80 percent of aesthetic intubation procedures involve the presence of secreted blood. It is known to breathing system filters in intensive care or anaesthesia endotracheal intubation procedures. The filters act as a barrier against infectious prions, and may be further improved by adding the NO eluting polymer technology to such a filter. In this way, breathing circuits and ventilators are protected more effectively against patient cross contamination. Such filters are disposable single use filters, have standard connectors 41, 42 for universal fit on standard breathing circuits, and a large pleated filter surface offers minimal breathing resistance. An example of such a filter 4 is shown in Fig. 4. According to an embodiment, the pleated filter surface 40 is coated with NO eluting polymers.

Such breathing circuit filters may also be used to stop the spread of SARS (severe acute respiratory syndrome). In 2003 an epidemic of SARS occurred in Toronto. The initial outbreak was characterized by nosocomial transmission. Nosocomial infections, in particular infections involving healthcare workers, have decreased, but they have not disappeared. Many of these infections appear to have occurred during procedures in intensive care units in which staff were exposed to a very high burden of respiratory secretions (high frequency oscillation, difficult intubations, bronchoscopy, non-invasive ventilation, aerosolized therapy etc.). The virus causing SARS mutated from corona virus family and is about 0.06-0.22µm in size. It cannot be filtered out with the expiratory isolation system which can retain bacteria of 0.3µm at 99.7% efficiency only. Therefore virus filters should be placed both on the inspiratory and expiratory end of the system to prevent staff contamination. Face or nose masks may also be used for patients not needing invasive ventilation. In both cases, the efficiency against SARS is improved by implementing NO eluting polymer into the filters or filtering masks.

Another topic is that the World Health Organisation (WHO) is fearing a killer flu pandemic, alarmed by the H5N1 strain of bird flu, which has become endemic in a number of Asian countries and which health officials fear could eventually mutate into a lethal new virus that will spread rapidly amongst humans.

The United Nations health agency, which sees a potential death toll of two million to seven million as a "best case scenario" for an outbreak. A more recent WHO estimate is that H5N1 could infect up to 30 percent of the world's population and kill maybe 20 to 50 million people. It will be incomparable to SARS, referring to the above-mentioned Severe Acute Respiratory Syndrome epidemic that killed 800 people around the world in 2003. While SARS had a mortality rate of around 15 percent, the deadly H5N1 strain of bird flu kills up to a third of the people it infects. H5N1 has proven to be versatile and is able to latch itself onto more hosts. It has gone through huge genetic changes and become more pathogenic.

WHO and influenza experts worldwide are concerned that the recent appearance and widespread distribution of an avian influenza -- Influenza A/H5N1 -- has the potential to ignite the next pandemic. WHO stated that it was impossible to predict just how deadly any such outbreak would be, because that would depend on various factors, including the virus' virulence and the ease of transmission. However, the global spread of a pandemic cannot be stopped, but preparedness will reduce its impact. The concern is not just about the dying, it is the hundreds of millions that will be sick and who are going to flood into hospitals.

With this background, the anti-pathogenic functionality added to conventional viral filters, by means of the NO eluting polymers, helps to stop such a pandemic more effectively.

Face masks belong to the group of personal protective equipment for breathing protection. Filtering Facepieces offer effective and economic cup shape protection. Gas masks comprising particle filters, gas filters, or combination filters are known. Respiratory protective filters are a low-cost and effective means of removing contaminants from breathing air. By adding the NO eluting functionality according to the present embodiment of the invention allows them to be even more effective for pathogenic and/or cancerous elements without degrading their conventional filter performance. The skilled person will appreciate that such filters are compatible with known face mask types, comprising half- and full face masks. An example for a gas mask combination filter 5 is shown in Fig. 5, wherein the filter is illustrated partly cut out. One section 51 of the combination filter 5 is to filter out gas, and the other section 52 is to filter out particles. NO eluting polymers may be added in either or both sections 51, 52.

Although the present invention has been described above with reference to specific embodiments, it is not intended to be limited to the specific form set forth herein. Rather, the invention is limited only by the accompanying claims and, other embodiments than the specific above are equally possible within the scope of these appended claims, e.g. different arrangements of fibres, or different polymers NO eluting polymers, or different filter forms, than those described above.

In the claims, the term "comprises/comprising" does not exclude the presence of other elements or steps. Furthermore, although individually listed, a plurality of means, elements or method steps may be implemented by e.g. a single unit. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A filtering device adapted to eliminate, inactivate and/or remove pathogenic and/or cancerous elements from fluids, especially body fluids or fluids getting into contact with the body, comprising
a nitric oxide (NO) eluting polymer arranged to contact said fluid when in use of said filtering device passing there through, such that a therapeutic dose of nitric oxide is eluted from said nitric oxide eluting polymer to said fluid.

2. The filtering device according to claim 1, wherein said pathogenic elements comprise microorganisms comprised in the group of bacteria, parasites, fungi, mycoplasma, protozoa, and viruses, and wherein said cancerous elements are tumour cells.

3. The filtering device according to claim 1 or 2, wherein said fluid is a is blood and said filtering device is comprised in an extracorporeal circuit configured to transport blood from a patient and/or after treatment of blood returns treated blood to the patient.

4. The filtering device according to claim 3, wherein said extracorporeal circuit is comprised in an extracorporeal blood treatment apparatus, particularly comprised in the group comprising a dialysis apparatus, an oxygenator, or arbitrary combinations thereof.

5. The filtering device according to claim 3, wherein said filtering device is an embolism filter.

6. The filtering device according to claim 1 or 2, wherein said body fluid is inspiratory or expiratory gas entering or leaving a mammal body through the mouth or nose thereof, wherein

7. The filtering device according to claim 1, wherein release of NO from said polymer is humidity triggered.

8. The filtering device according to any preceding claim, wherein said polymer is linear poly(ethylenimine) (L-PEI).

9. The filtering device according to claim 1, wherein said polymer comprises nanofibers arranged as or on a filter medium of said filtering device.

10. The filtering device according to claim 9, wherein of said nanofibers comprise poly(ethylenimine)-diazeniumdiolate.

11. A process for forming a filtering device adapted to eliminate, inactivate and/or remove pathogenic elements, especially microorganisms, such as bacteria, parasites, protozoa and viruses, or cancerous elements from fluids, comprising
selecting a plurality of nitric oxide eluting polymeric fibers, and
deploying said nitric oxide eluting fibers to be comprised in a filter membrane.

12. The process according to claim 11, wherein said deploying of fibres comprises electrospinning of nanofibers of poly(ethylenimine)-diazeniumdiolate, comprising depositing of said nanofibers on said filtering device.

13. A method for eliminating, inactivating and/or removing pathogenic elements, especially microorganisms, such as bacteria, parasites, fungi, mycoplasma, protozoa and viruses, or cancerous elements from fluids, comprising
providing a filtering device comprising a nitric oxide eluting polymer, and
eluting nitric oxide from the polymer upon contact with the fluid for achieving an anti-pathogenic and/or anti-cancerous effect.

14. Use of nitric oxide (NO) for adding anti-pathogenic and/or anti-cancerous functionality to a medical filtering device.

15. Use of nitric oxide (NO) eluting polymers for elution of NO from a medical filtering device for adding anti-pathogenic and/or anti-cancerous functionality to the medical filtering device.
